# EUROPÄISCHE PATENTANMELDUNG

(11) **EP 1 712 544 A1**
(43) Veröffentlichungstag der Anmeldung: **18.10.2006**
(21) Anmeldenummer: 05008038.1
(22) Anmeldetag: 12.04.2005
(51) Int. Cl.: C07C 231/04, C07C 235/80

(54) **Verfahren zur Herstellung von Acetessigsäurearylamiden**

(71) Anmelder: Lonza AG, 4052 Basel (CH)
(72) Erfinder: Armbruster, Erich, 3904 Naters (CH); Quach, Thuy, 3902 Glis (CH); Rosenthal, Günther, 3900 Brig (CH); Schwegler, Brian, 3930 Visp (CH)

(57) **Zusammenfassung**

Acetessigsäurearylamide werden aus Diketen und primären oder sekundären aromatischen Aminen unter den Bedingungen einer Reaktivdestillation in Gegenwart von Wasser hergestellt. Das kontinuierliche Verfahren zeichnet sich durch hohe Reinheit der Produkte, fast quantitative Ausbeuten und hohe Durchsatzraten aus.

## Beschreibung

Die Erfindung betrifft ein kontinuierliches Verfahren zur Herstellung von Acetessigsäurearylamiden aus Diketen und primären oder sekundären aromatischen Aminen.

Acetessigsäurearylamide sind wichtige Ausgangsprodukte zur Herstellung von Farbstoffen und Pigmenten, dienen aber auch zur Herstellung von agrochemischen Wirkstoffen (siehe z.B. Ullmann's Encyclopedia of Industrial Chemistry, 5th Ed., Vol. 15, p. 71).

Die Herstellung von Acetessigsäurearylamiden ist seit langem bekannt und basiert auf der Umsetzung von Diketen mit entsprechenden aromatischen Aminen in verschiedenen organischen und/oder wässrigen Lösungsmitteln und Lösungsmittelgemischen (Ullmann's Encyclopedia of Industrial Chemistry, 5th Ed., Vol. 15, p. 71).
In der Regel wird hierbei diskontinuierlich in Wasser oder wässrigen Lösungen gearbeitet Dementsprechend offenbart DE-A-25 19 036 die Herstellung von verschiedenen Acetessigsäurearylamiden durch simultane Zudosierung von Diketen und dem entsprechenden aromatischen Amin in Gegenwart von Wasser oder wässrigen Lösungen. Das resultierende Acetessigsäurearylamid wird im Reaktionsmedium gekühlt und auskristallisieren gelassen. Nach Zentrifugation und Trocknung werden die Acetessigsäurearylide in guter Ausbeute und hoher Reinheit erhalten.

Aus EP-A-0 648 738 ist ausserdem ein kontinuierliches Verfahren zur Herstellung von Acetessigsäurearylamiden bekannt. Darin wird Diketen kontinuierlich mit dem aromatischen Amin in einem Gemisch aus Wasser und Alkohol unter möglichst langer Verweilzeit des Reaktionsgemisches im Reaktor hergestellt. Das resultierende Acetessigsäurearylamid wird durch Kristallisation des aus dem Reaktor ausgeschleusten Produktstroms gewonnen.

In FP-A-0 945 430 ist ein Verfahren zur Herstellung wasserhaltiger Acetessigsäurearylamide aus Diketen und aromatischen Aminen offenbart, wobei sich im Verlauf der Reaktion ein Zweiphasensystem aus Produktschmelze und einer wässrigen Phase bildet. Das Verfahren eignet sich für die ansatzweise, halbkontinuierliche oder kontinuierliche Durchführung und liefert ausreichend reine Produkte in guten Ausbeuten, erfordert aber ziemlich lange Verweilzeiten bzw. ― wegen der stark exothermen Reaktion ― im Verhältnis zum Durchsatz relativ grosse Reaktorinhalte ("hold up"). So wurden in einem Rohrreaktor von 10 cm Durchmesser nur Produktdurchsätze von ca. 1,5 kg/h erhalten. Ausserdem erfordert das Verfahren relativ grosse Wassermengen und den Einsatz von Diketen im Überschuss, um Verluste durch Neben- und Folgereaktionen zu kompensieren. Aufgabe der vorliegenden Erfindung war daher die Bereitstellung eines alternativen Verfahrens, das ebenfalls reine Produkte in sehr guter Ausbeute liefert und sich insbesondere für die kontinuierliche Durchführung bei hohen Durchsatzraten eignet und mit annähems stöchiometrischen Mengen von Diketen auskommt.

Die Aufgabe konnte erfindungsgemäss gelöst werden, indem die Reaktion von Diketen und aromatischem Amin nach Art einer Reaktivdestillation in Gegenwart von Wasser durchgeführt wird. Die Reaktionswärme wird hierbei durch die Verdampfung des Wassers absorbiert und über den Kondensator abgeführt. Da bei der Reaktion im Idealfall keine Nebenprodukte entstehen, sich kein Gleichgewicht zwischen Edukten und Produkt einstellt, das Produkt schwerer flüchtig ist als die Edukte Diketen und Amin und deshalb als Sumpfprodukt anfällt und das Kopfkondensat im wesentlichen aus dem zugesetzten Wasser besteht und das Rücklaufverhältnis deshalb sehr hoch sein kann, handelt es sich nicht um eine Reaktivdestillation im engeren Sinn. Die verwendbaren Apparaturen entsprechen jedoch weitgehend denjenigen, die für die klassischen Reaktivdestillationen eingesetzt werden, weshalb hier der Ausdruck "nach Art einer Reaktivdestillation" gebraucht wird. Hierunter ist im einzelnen zu verstehen, dass gleichzeitig eine chemische Reaktion in der Flüssigphase und ein thermischer Stoffaustausch zwischen der Flüssigphase und einer Dampfphase im Gegenstrom stattfindet.

Das erfindungsgemässe Verfahren eignet sich grundsätzlich für die Herstellung aller Acetessigsäurearylamide, die bei den üblichen Reaktionstemperaturen flüssig sind oder mit Wasser flüssige Schmelzen bilden.

Vorzugsweise besitzen die erfindungsgemäss herstellbaren Acetessigsäurearylamide die allgemeine Formel 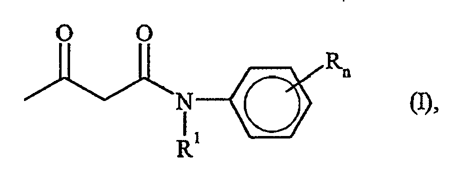 worin R¹ Wasserstoff oder eine C₁₋₆-Alkylgruppe und n eine ganze Zahl von 0 bis 5 ist und die Substituenten R, soweit vorhanden, gleich oder verschieden und aus der Gruppe bestehend aus C₁₋₆-Alkylgruppen, C₁₋₆-Alkoxygruppen und Halogenatomen ausgewählt sind.

Unter C₁₋₆-Alkylgruppen sind hier alle linearen oder verzweigten Alkylgruppen mit 1 bis 6 Kohlenstoffatomen zu verstehen, insbesondere Methyl, Ethyl, *n*-Propyl, Isopropyl, *n*-Butyl, Isobutyl, *sec*-Butyl, *tert*-Butyl, *n*-Pentyl, Isopentyl, *tert*-Pentyl, Neopentyl, *n*-Hexyl usw. Bevorzugtes Alkyl ist Methyl. Unter C₁₋₆-Alkoxygruppen sind die aus den vorstehend genannten C₁₋₆-Alkylgruppen und Sauerstoff zusammen gesetzten Gruppen zu verstehen. Bevorzugt ist hier Methoxy. Entsprechendes gilt für die analog gebildeten Ausdrücke "C₁₋₄-Alkyl" und "C₁₋₄-Alkoxy". Halogen steht für Fluor, Chlor, Brom oder Iod, bevorzugt für Chlor.

Besonders bevorzugt ist die Herstellung solcher Acetessigsäurearylamide der Formel I, in denen R¹ Wasserstoff ist.

Ebenfalls besonders bevorzugt ist die Herstellung solcher Acetessigsäurearylamide der Formel I, in denen n eine ganze Zahl von 0 bis 3 ist und die Substituenten R, soweit vorhanden, unabhängig voneinander aus der Gruppe bestehend aus C₁₋₄-Alkylgruppen, C₁₋₄-Alkoxygruppen und Chlor ausgewählt sind.

Ganz besonders bevorzugte Acetessigsäurearylamide leiten sich von Anilin (n = 0), *o*-Anisidin (n = 1; R = *o*-Methoxy), *o*-Toluidin (n = 1; R = *o*-Methyl), den Xylidinen (n = 2; R = Methyl), *o*-Chloranilin (n = 1; R = *o*-Cl), 2,4-Dimethoxyanilin (n = 2; R = 2,4-Dimethoxy), 4-Isopropylanilin (n = 1; R = *p*-Isopropyl), 4-Ethoxyanilin (n = 1; R =*p*-Ethoxy), 2,5-Dimethoxyanilin (n = 2; R = 2,5-Dimethoxy) und 4-Chlor-2,5-dimethoxyanilin (n = 3; R = 4-Cl, 2,5-Dimethoxy) ab.

Durch Wahl eines entsprechenden Drucks kann die Verdampfungstemperatur des Wassers und damit die Reaktionstemperatur im für das jeweilige Produkt günstigsten Bereich gehalten werden. Die Reaktionstemperatur wird zweckmässig so gewählt, dass das Produkt in flüssiger Form anfällt, aber thermisch möglichst wenig belastet wird. Vorzugsweise wird das erfindungsgemässe Verfahren unter vermindertem Druck durchgeführt.

Zur Durchführung des erfindungsgemässen Verfahrens eignen sich insbesondere Bodenkolonnen wie beispielsweise Schlitzbodenkolonnen mit geringem Druckverlust. Es ist aber auch möglich, gepackte Kolonnen zu verwenden.

In einer besonders bevorzugten Ausführungsform werden zwei Kolonnen verwendet, wobei in der einen Kolonne die eigentliche Reaktion stattfindet und in der anderen Kolonnen das Sumpfprodukt der ersten Kolonne mit Wasserdampf von überschüssigen Edukten und anderen flüchtigen Verunreinigungen wie beispielsweise Aceton befreit ("gestrippt") wird.

Die erfindungsgemäss hergestellten Acetessigsäurearylamide fallen in Form einer wasserhaltigen Schmelze an. Diese kann mittels in der Fachwelt üblicher Methoden in feste Formen wie beispielsweise Pastillen, Schuppen oder Prillen übergeführt werden. Die Dimension und Grösse der genannten Formen ist abhängig von der Ausgestaltung der Pastillier-, Schupp- oder Prillierverfahren und kann in einem weiten Bereich variieren. Die so erhaltenen Acetessigsäurearylamide weisen insbesondere eine gute Rieselfähigkeit und ein vorteilhaftes Lösungsverhalten auf Wird ein wasserfreies Produkt gewünscht, so kann ein erfindungsgemäss erhaltenes Acetessigsäurearylamid auf herkömmliche Weise getrocknet werden.

### Beschreibung der Zeichnungen:

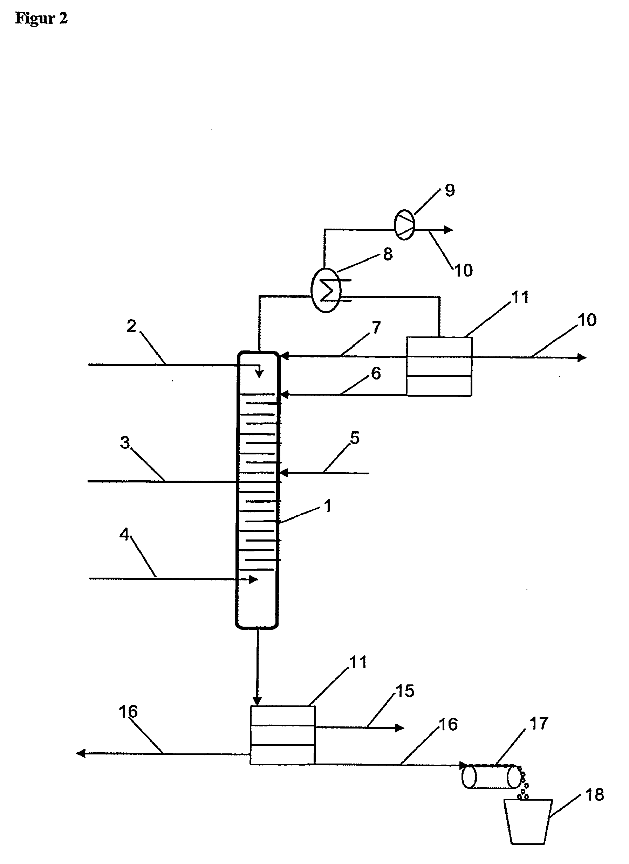

Die Zeichnungen haben lediglich beispielhaften Charakter und sind nicht als Einschränkungen zu verstehen.

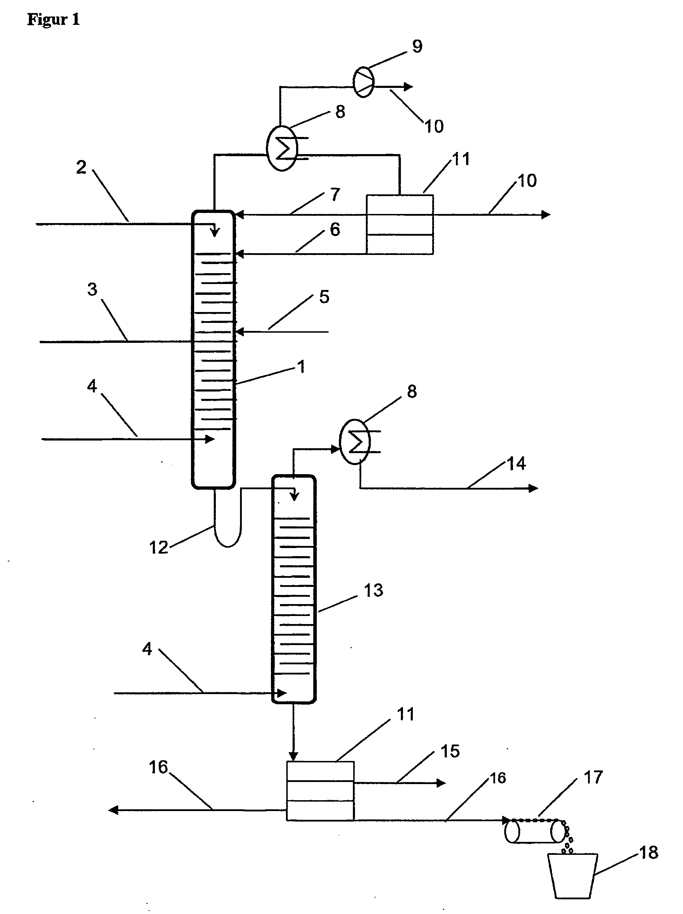

Figur 1 stellt eine Anordnung für das erfindungsgemässe Verfahren mit zwei Kolonnen dar. Die Bezugszeichen bedeuten im Einzelnen:
   1 Reaktionskolonne
   2 Anilineinspeisung
   3 Diketeneinspeisung
   4 Dampfeinspeisung
   5 Wassereinspeisung
   6 Kondensatrücklauf organische (schwere) Phase
   7 Kondensatrücklauf Wasserphase
   8 Kondensator
   9 Vakuumpumpe
   10 Abgas (zur Abgasbehandlung)
   11 Phasenabscheider
   12 Sumpfprodukt zur Strippkolonne
   13 Strippkolonne
   14 Kondensatablauf (zur Abwasserbehandlung)
   15 Ablauf Wasserphase (zur Abwasserbehandlung)
   16 Produktaustrag (flüssig)
   17 Kristallisierband ("Schupper")
   18 Vorlage für erstarrtes Produkt
Figur 2 stellt eine vereinfachte Anordnung ohne Strippkolonne dar. Die Bedeutung der Bezugszeichen ist die gleiche wie in Figur 1.

Die folgenden Beispiele verdeutlichen die Durchführung des erfindungsgemässen Verfahrens, ohne dass darin eine Einschränkung zu sehen ist.

### Beispiel 1

### Herstellung von Acetessigsäureanilid

Eine Destillationskolonne (ID = 100mm) mit 35 Schlitzböden ("slit tray", Firma Kühni AG, Allschwil, Schweiz) wurde unter Teilvakuum (220 mbar Kopfdruck) mit Wasser wie folgt eingefahren:
Wasserdampf-Einspeisung Mitte (unterhalb Boden 17): 6 kg/h
Wasserdampf-Einspeisung unten: 6 kg/h
Die Kolonne arbeitete unter totalem Rückfluss.
Nachdem sich der Differenzdruck (65―70 mbar) und das Temperaturprofil (65―70 °C) stabilisiert hatten, wurde begonnen, in den Kolonnenkopf Anilin und auf Boden 14 Diketen einzuspeisen, wobei die Einspeisemenge alle 10-15 Minuten in Schritten von ca. 1 kg/h bis auf folgende Endwerte erhöht wurde:
Anilin: 0,115 kmol/h (= 10,7 kg/h)
Diketen: 0,117 kmol/h (= 9,83 kg/h)
Gleichzeitig wurde die Wasserdampfeinspeisung oben (unterhalb Boden 17) in simultanen Schritten um jeweils ca. 0,5 kg/h auf schliesslich 1 kg/h reduziert. Um sicherzustellen, dass in der Reaktionszone beim Hochfahren genügend Wasser für die Abfuhr der Reaktionswärme zur Verfügung steht, wurde zudem auf Boden 14 Wasser mit 3 kg/h eingespeist. Die zunehmende Belastung der Kolonne wurde durch Reduktion der unteren Dampfeinspeisung auf 3 kg/h kompensiert.
Sobald sich in der Kolonne nach Erreichen der Sollwerte für die Einspeisemengen ein stationärer Zustand eingestellt hatte (Differenzdruck 70-75 mbar, Temperatur 60-70 °C) wurde die im Sumpf anfallende Schmelze von Acetessigsäureanilid über einen Phasenabscheider vom überschüssigen Wasser getrennt und auf einem Trommelschupper zum Erstarren gebracht. Das Produkt fiel in Form wasserfeuchter weisser Schuppen an, die einen Gehalt von 90% Acetessigsäureanilid und 10% Wasser aufwiesen. Die Summe aller nicht aus Edukt-Verunreinigungen stammenden Nebenprodukte lag unter 0,05 Flächenprozent (HPLC).

### Beispiel 2

### Herstellung von Acetessigsäure-m-xylidid

Eine Destillationskolonne (ID = 100mm) mit 17 Schlitzböden der gleichen Bauart wie in Beispiel 1 (Reaktionskolonne) wurde unter Teilvakuum (370 mbar Kopfdruck) von unten mit Wasserdampf (6 kg/h) und auf Boden 14 mit Wasser (6 kg/h) beaufschlagt.
Gleichzeitig wurde eine zweite Destillationskolonne (ID = 100mm) mit 18 Schlitzböden gleicher Bauart (Strippkolonne) unter Teilvakuum (370 mbar Kopfdruck) mit Wasserdampf (11 kg/h) eingefahren.
Beide Kolonnen arbeiteten mit separaten Kondensatoren und zunächst unter totalem Rückfluss. Die erste Kolonne war mit einer Blase (Volumen ca. 6 L) für das Sumpfprodukt versehen, der Blasenüberlauf wurde auf den Kopf der zweiten Kolonne geleitet.
Nachdem sich in beiden Kolonnen der Differenzdruck (25-35 mbar) und das Temperaturprofil (75-81 °C) stabilisiert hatten wurde auf den Kopf der ersten Kolonne *m*-Xylidin (2,4-Dimethylanilin) (6,4 kg/h) und auf deren vierzehnten Boden Diketen (4,7 kg/h) eingespeist. Dabei wurde die Diketeneinspeisung erst gestartet, nachdem der xylidinhaltige Rücklauf den Boden 14 erreicht hatte. Die Einspeisemengen der Edukte wurden unter Einhaltung des stöchiometrischen Verhältnisses langsam hochgefahren, bis nach ca. einer Stunde die gewünschte Leistung erreicht wurde:
Anilin 0,115 kmol/h (= 14,0 kg/h)
Diketen 0,122 kmol/h (= 10,3 kg/h)
Gleichzeitig wurde die Wasserzufuhr auf Boden 14 auf 1,8 kg/h verringert.
Das Kondensat aus der zweiten Kolonne wurde nicht mehr zurückgeführt, um das überschüssige Diketen aus dem System zu entfernen.
Sobald sich die Kolonnen nach Erreichen der Sollwerte für die Einspeisung stabilisiert hatten (Differenzdruck je ca. 25-30 mbar, Temperaturen 76-78 °C) wurde die im Sumpf der zweiten Kolonne anfallende Acetessigsäure-*m*-xylidid-Schmelze durch einen Phasenabscheider geleitet, um eventuell überschüssiges Wasser abzutrennen, und auf einem Trommelschupper zum Erstarren gebracht. Das Produkt fielt in Form wasserfeuchter weisser Schuppen an, die einen Gehalt von 92,1 % Acetessigsäure-*m*-xylidid und 7,7% Wasser aufweisen. Der Umsatz (bezogen auf das eingesetzte *m*-Xylidin) war innerhalb der geschätzten Fehlergrenzen (±0,5%) quantitativ (99,8%). Der Diketenüberschuss wurde mit dem Kondensat der zweiten Kolonne fast quantitativ entfernt. Das Produkt enthielt als Verunreinigung nur noch ca. 0,2% Aceton (aus Diketen durch Hydrolyse und Decarboxylierung).

### Beispiel 3

### Herstellung von Acetessigsäure-m-xylidid

Eine Destillationskolonne (ID = 100mm) mit 22 Schlitzböden der in Beispiel 1 beschriebenen Art (Reaktionskolonne) wurde unter Teilvakuum (355 mbar Kopfdruck) mit Dampf (5 kg/h) eingefahren.
Gleichzeitig wurde eine zweite Destillationskolonne (ID = 100mm) mit 13 Schlitzböden gleicher Bauart (Strippkolonne) unter Teilvakuum (355 mbar Kopfdruck) mit Dampf (5 kg/h) eingefahren.
Beide Kolonnen arbeiteten mit separaten Kondensatoren, zunächst unter totalem Rückfluss. Die erste Kolonne war mit einer Blase (ca. 8 L) für das Sumpfprodukt versehen, der Blasenüberlauf wurde in den Kopf der zweiten Kolonne geleitet.
Nachdem sich der Differenzdruck (Kolonne 1: ca. 45 mbar, Kolonne 2: ca. 25 mbar) und das Temperaturprofil (73-77 °C) stabilisiert hatten wurde auf den zweiten Boden der ersten Kolonne *m*-Xylidin (16,9 kg/h) und auf die Böden 14, 15 und 16 verteilt Diketen (total 12,0 kg/h) eingespeist. Dabei wurde die Diketeneinspeisung erst gestartet, nachdem der xylidinhaltige Rücklauf den Boden 14 erreicht hatte.

Ein Teil des Kondensates der ersten Kolonne wurde entnommen (ca. 1 kg/h), um eine Anreicherung des im Diketen vorhandenen Acetons zu vermeiden. Das Kondensat aus der zweiten Kolonne wurde nicht mehr zurückgeführt, um das überschüssige Diketen aus dem System zu entfernen.
Sobald sich die Kolonnen stabilisiert hatten (Differenzdruck 1. Kolonne ca. 50 mbar, zweite Kolonne ca. 25-30 mbar, Temperaturen 74-77 °C), wurde die im Sumpf der zweiten Kolonne anfallende Acetessigsäure-*m*-xylidid-Schmelze in einem Phasenabscheider vom überschüssigen Wasser getrennt und auf einem Trommelschupper zum Erstarren gebracht. Das Produkt fiel in Form wasserfeuchter weisser Schuppen an, die einen Gehalt von 91,5% Acetessigsäure-*m-*xylidid und ca. 8,5% Wasser aufwiesen. Der Umsatz bezogen auf das eingesetzte *m*-Xylidin war innerhalb der geschätzten Fehlergrenzen (±0,8%) quantitativ (100%). Der Diketenüberschuss wurde mit dem Kondensat der zweiten Kolonne fast quantitativ entfernt. Das Produkt enthielt nur noch ca. 0,1 % Aceton (aus Diketen durch Hydrolyse und Decarboxylierung).

## Patentansprüche

1. Kontinuierliches Verfahren zur Herstellung von Acetessigsäurearylamiden aus Diketen und primären oder sekundären aromatischen Aminen, **dadurch gekennzeichnet, dass** die Reaktion von Diketen mit dem aromatischen Amin in Gegenwart von Wasser nach Art einer Reaktivdestillation durchgeführt wird.

2. Verfahren nach Anspruch 1, **dadurch gekennzeichnet, dass** die Acetessigsäurearylamide die allgemeine Formel 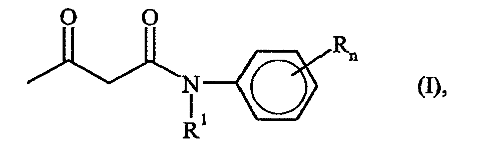 worin R¹ Wasserstoff oder eine C₁₋₆-Alkylgruppe und n eine ganze Zahl von 0 bis 5 ist und die Substituenten R, soweit vorhanden, unabhängig voneinander aus der Gruppe bestehend aus C₁₋₆-Alkylgruppen, C₁₋₆-Alkoxygruppen und Halogenen ausgewählt sind, besitzen und aus Diketen und einem primären oder sekundären aromatischen Amin der allgemeinen Formel 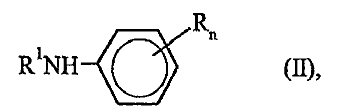 worin R¹, n und R die oben genannte Bedeutung haben, hergestellt werden.

3. Verfahren nach Anspruch 2, **dadurch gekennzeichnet, dass** R¹ Wasserstoff ist.

4. Verfahren nach Anspruch 2 oder 3, **dadurch gekennzeichnet, dass** n eine ganze Zahl von 0 bis 3 ist und die Substituenten R, soweit vorhanden, unabhängig voneinander aus der Gruppe bestehend aus C₁₋₄-Alkylgruppen, C₁₋₄-Alkoxygruppen und Chlor ausgewählt sind.

5. Verfahren nach einem der Ansprüche 1 bis 4, **dadurch gekennzeichnet, dass** die Reaktivdestillation unter vermindertem Druck durchgeführt wird.

6. Verfahren nach einem der Ansprüche 1 bis 5, **dadurch gekennzeichnet, dass** die Reaktion in wenigstens einer Bodenkolonne durchgeführt und das Acetessigsäurearylamid als Sumpfprodukt erhalten wird.

7. Verfahren nach einem der Ansprüche 1 bis 6, **dadurch gekennzeichnet, dass** es in zwei Kolonnen durchgeführt wird, wobei die Reaktion in der einen Kolonne stattfindet und das Sumpfprodukt dieser Kolonne in einer zweiten Kolonne mit Wasserdampf von überschüssigen Edukten und flüchtigen Verunreinigungen befreit (gestrippt) wird.

8. Verfahren nach einem der Ansprüche 1 bis 7, **dadurch gekennzeichnet, dass** das gegebenenfalls gestrippte flüssige Sumpfprodukt, nach Abtrennung einer gegebenenfalls abgeschiedenen Wasserphase, in Form von Schuppen, Pastillen oder Prills verfestigt wird.
